# EUROPEAN PATENT APPLICATION

(11) **EP 4 768 041 A1**
(43) Date of publication of application: **01.07.2026**
(21) Application number: 24864785.1
(22) Date of filing: 14.09.2024
(51) Int. Cl.: A61K 38/20, A61K 47/60, A61P 35/00

(54) **COMBINATION THERAPY FOR TREATING TUMORS AND USE THEREOF**

(30) Priority: 15.09.2023 CN 202311192714
(71) Applicant: Jenkem Technology Co., Ltd. (Beijing), Haidian District Beijing 100192 (CN)
(72) Inventor: WANG, Qingbin, Beijing 100192 (CN); LIU, Biao, Beijing 100192 (CN); LI, Qiufen, Beijing 100192 (CN); ZHENG, Xichun, Beijing 100192 (CN); WANG, Jie, Beijing 100192 (CN); XIA, Ke, Beijing 100192 (CN); GUO, Jun, Beijing 100192 (CN); ZHAO, Xuan, Beijing 100192 (CN)
(74) Representative: HGF
(86) International application number: PCT/CN2024/119183
(87) International publication number: WO 2025/056070

(57) **Abstract**

Provided are a combination therapy for treating tumors and a use thereof. The combination therapy comprises combined use of polyethylene glycol-modified IL-2 and radiation. The use of the combination therapy can effectively reduce the tumor volume and increase the tumor growth inhibition rate, and has a significantly improved effect compared with polyethylene glycol-modified IL-2 monotherapy or radiation alone.

## Description

### TECHNICAL FIELD

The present disclosure relates to the technical field of pharmaceuticals, and particularly relates to a combination therapy for treating a tumor and use thereof.

### BACKGROUND

In recent years, both the morbidity and mortality of cancers have maintained an upward trend. The age of onset of cancers also tends to be younger, and most patients are already in the middle or late stages at the time of diagnosis. Therefore, reducing the morbidity and mortality of cancers is an urgent public health problem to be solved, and the development of new potent antitumor therapeutic methods is still the main direction for current tumor therapy.

Interleukin 2 (IL-2) is a cytokine derived from multiple cells, having a pleiotropic effect, and it has an important effect on the immune response, virus infection resistance, etc., of organisms. IL-2 can activate T cells and stimulate the proliferation of T cells that are initiated by specific antigens, promoting the production of cytokines. It stimulates NK cell proliferation, enhances NK cell killing activity and cytokine generation, and induces the generation of LAK cells. Also, it promotes B cell proliferation and antibody secretion, and activates macrophages.

Because IL-2 can induce and enhance cytotoxic activity, research on the use of IL-2 for treating certain diseases, particularly for treating tumors, is widely carried out. The use of IL-2 alone or in combination with LAK cells and the like has achieved certain therapeutic effects in treating tumors, and IL-2 is also expected to be used in treating viral infections, immunodeficiency diseases, and autoimmune diseases. The recombinant IL-2 drug Proleukin (Aldesleukin), jointly developed by Novartis and linigen, was approved by the FDA for treating metastatic renal cancer in 1991, and it was approved for treating metastatic melanoma in 1998. However, due to its short half-life, narrow therapeutic window, significant toxic and side effects, and simultaneous stimulation of Treg activation, its clinical use is limited. In order to improve the pharmacokinetic properties and reduce toxic and side effects of IL-2, researchers change the affinity of IL-2 for different receptor subunits by means of amino acid mutation or chemical modification to increase activity and reduce toxicity. In addition, the prior art also discloses the use of IL-2 in combination with other tumor treatment methods to improve the therapeutic effect. For example:
Patent document CN114767835A discloses a pharmaceutical composition for treating colorectal cancer and use thereof. An active ingredient of the pharmaceutical composition comprises or consists of: polyethylene glycol-modified IL-2 and capecitabine. The combination of the polyethylene glycol-modified IL-2 and capecitabine can be used for treating colon cancer. This technology combines the IL-2 with chemotherapy for treating a cancer.

Tumor radiation therapy is a local treatment method using radiation to treat tumors. The radiation includes α, β, and γ rays produced by a radioisotope, and an X-ray produced by an X-ray therapy machine or an accelerator, as well as an electron beam, fast neutrons, a proton beam, a negative π-meson beam, a heavy particle beam, or the like. About 70% of cancer patients require radiation therapy during the treatment of a cancer, and about 40% of cancers can be radically cured with radiotherapy. The role and status of radiation therapy in the tumor treatment are becoming increasingly prominent, and radiation therapy has become one of the main means for treating malignant tumors.

However, although radiotherapy can also radically cure tumors, high-dose radiotherapy or long-term radiotherapy can also cause many side effects, such as increased intracranial pressure, nausea, vomiting, headache, itchy skin, dry skin, hair loss, reduced white blood cell count, reduced platelet activity, dyspnea, hemorrhage of mucosal tissues in the oral cavity or intestinal tract, and redness and swelling.

### SUMMARY

In order to solve the problems of the prior art, the present disclosure provides PEG-modified IL-2 in combination with radiotherapy for treating a specific solid tumor (especially gastrointestinal tumor, lung cancer, lymphoma, skin cancer, or sarcoma). A polyethylene glycol modification is performed on a binding site in the IL-2 that binds to an IL-2 receptor α subunit, thereby changing the affinity of IL-2 for different receptor subunits. The PEG-modified IL-2 stimulates the proliferation of tumor-killing T cells by targeting CD8⁺ effector T cells, while simultaneously attenuating the immunosuppression caused by Tregs, thereby utilizing the patient's own immune system to fight against the cancer. The radiotherapy mainly destroys the DNA structure of tumor cells via radiation, thereby inhibiting the proliferation and growth of the tumor cells and achieving the effect of alleviating the disease condition. The mechanisms of action of the two treatment modalities can complement each other to achieve a synergistic therapeutic effect, for example, greatly extending the *in vivo* half-life, effectively prolonging the duration of action, and reducing toxic and side effects. Moreover, the total dose, single dose, or frequency of radiotherapy in the combination therapy is greatly reduced, effectively alleviating side effects and pain of the patient. The specific technical solutions are as follows:
In a first aspect of the present disclosure, provided is use of polyethylene glycol-modified IL-2 in combination with radiation in preparing a product for treating a tumor.

Preferably, the polyethylene glycol-modified IL-2 has a structure of formula (I) as follows: 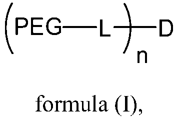
wherein PEG represents a polyethylene glycol residue;
L represents a linking group between PEG and D;
D represents a residue of IL-2;
n is an integer of 1-12, and preferably, n is an integer of 1-7, such as 1, 2, 3, 4, 5, 6, 7, 8, 9, 10, 11, or 12.

Preferably, L has the following structure: 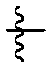 L₁-L₂-L₃ 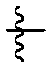 , wherein L₁ is selected from: a single bond, C1-6 alkylene, -(C1-6 alkylene)-NR^{L1}-, -(C1-6 alkylene)-O-, -(C1-6 alkylene)-S-, -(C1-6 alkylene)-NR^{L1}CO-, -(C1-6 alkylene)-CONR^{L1}-, -(C1-6 alkylene)-CO-, -(C1-6 alkylene)-OCO-, - (C1-6 alkylene)-NHCONR^{L1}-, -(C1-6 alkylene)-SO₂-, and -(C1-6 alkylene)-SO-; L₂ is selected from: a single bond, C1-6 alkylene, cycloalkylene, arylene, and heterocyclylene; L₃ is selected from: C1-6 alkylene, -(C1-6 alkylene)-NRL²-, -(C1-6 alkylene)-NR^{L2}CO-, -(C1-6 alkylene)-CO-, and -(C1-6 alkylene)-OCO-; R^{L1} and R^{L2} are independently selected from: H and C1-6 alkyl.

In some embodiments of the present disclosure, R^{L1} and R^{L2} are both H.

In some embodiments of the present disclosure, L₃ is -(C1-6 alkylene)-CO-.

In some embodiments of the present disclosure, L₂ is a single bond.

In some embodiments of the present disclosure, L₁ is selected from: a single bond, -(C1-6 alkylene)-NH-, -(C1-6 alkylene)-NHCO-, -(C1-6 alkylene)-CONH-, -(C1-6 alkylene)-CO-, and - (C1-6 alkylene)-OCO-.

In some embodiments of the present disclosure, L is -(C1-6 alkylene)-CO-, particularly -CH₂CH₂-CO-.

Preferably, the IL-2 is native IL-2, recombinant IL-2, or a mutant also having a native IL-2 function. Specifically, a linking site of a polyethylene glycol modification on the IL-2 is an amino (or amine) group, such as one or two or more of an N-terminal amino group of a peptide chain, a side chain amino group of a lysine residue within a peptide chain, a side chain amino group of an arginine residue, a secondary amine group on an imidazole ring of a histidine residue, and a secondary amine group on an indole ring of a tryptophan residue, particularly a side chain amino group of a lysine residue within a peptide chain.

Preferably, the number of the linking sites is one or two or more.

In one specific embodiment of the present disclosure, the number of the linking sites is two.

Preferably, the polyethylene glycol modification is performed on a binding site in the IL-2 that binds to an IL-2 receptor α subunit.

It should be understood that the polyethylene glycol-modified IL-2 means that the binding site in the IL-2 that binds to the IL-2 receptor α subunit is modified with polyethylene glycol.

Preferably, PEG has a molecular weight of 300-100000 daltons, further preferably 10000-60000 daltons, such as 300, 500, 1000, 5000, 10000, 15000, 20000, 25000, 30000, 35000, 40000, 45000, 50000, 55000, 60000, 65000, 70000, 75000, 80000, 85000, 90000, 95000, or 100000 daltons. Specifically, PEG may be a linear, Y-shaped, or multi-branched (e.g., four-, six-, or eight-arm) polyethylene glycol residue.

In one embodiment of the present disclosure, PEG has a structure represented by formula (II) as follows: 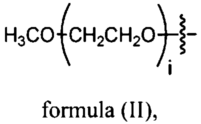
wherein i is an integer of 5-2500 (e.g., 5, 10, 20, 30, 40, 50, 100, 200, 300, 400, 500, 600, 700, 800, 900, 1000, 1100, 1200, 1300, 1400, 1500, 1600, 1700, 1800, 1900, 2000, 2100, 2200, or 2500). In another embodiment of the present disclosure, PEG has a structure represented by formula (III) or formula (IV) as follows: 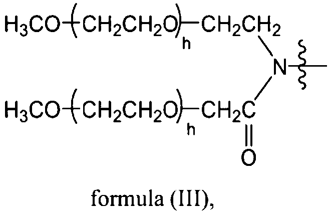 or 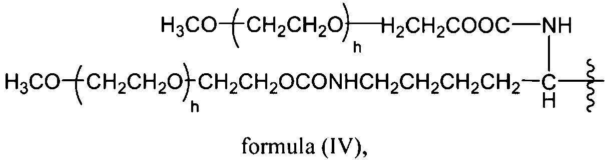
wherein h is an integer of 3-1200 (e.g., 3, 5, 10, 20, 30, 40, 50, 100, 150, 200, 250, 300, 350, 400, 450, 500, 550, 600, 650, 700, 800, 900, 1000, 1100, or 1200).

In another embodiment of the present disclosure, the PEG described above has a structure represented by formula (V) as follows: 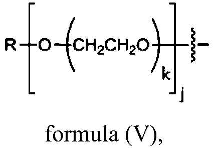 wherein:
k is an integer of 1-800 (e.g., 1, 5, 10, 20, 30, 40, 50, 100, 150, 200, 250, 300, 350, 400, 450, 500, 550, 600, 650, 700, 750, or 800);
j is an integer of 3-12 (e.g., 3, 4, 5, 6, 7, 8, 9, 10, 11, or 12);
R is a core molecule of multi-branched polyethylene glycol, and R may be selected from: residues of pentaerythritol, methyl glucoside, sucrose, diethylene glycol, propylene glycol, glycerol, and polyglycerol.

In some embodiments of the present disclosure, PEG has a structure represented by formula (VI) as follows: 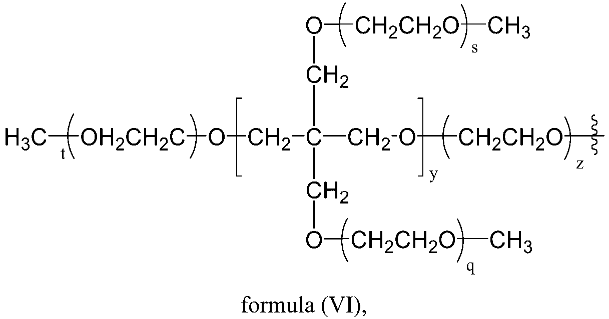
wherein q, s, t, and z are independently selected from an integer of 2-340 (e.g., 2, 5, 10, 20, 30, 40, 50, 60, 70, 80, 90, 100, 150, 200, 250, 300, 310, 320, 330, or 340),
and y is an integer of 1-5 (e.g., 1, 2, 3, 4, or 5).

In other embodiments of the present disclosure, PEG has a structure represented by formula (VII) as follows: 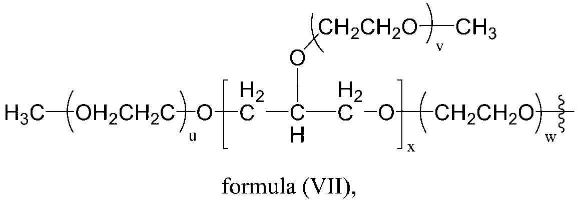
wherein u, v, and w are independently selected from an integer of 2-460 (e.g., 2, 5, 10, 50, 100, 150, 200, 250, 300, 350, 400, 450, or 460),
and x is an integer of 1-5 (e.g., 1, 2, 3, 4, or 5).

In some embodiments of the present disclosure, the polyethylene glycol-modified IL-2 has a structure of formula (VIII) as follows: 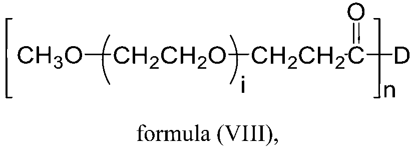 wherein n may be 1, 2, 3, or 4.

In one specific embodiment of the present disclosure, n is 2.

Specifically, a molecular weight of the polyethylene glycol residue in the polyethylene glycol-modified IL-2 represented by formula VIII may be 10000-60000 daltons, such as 20000 daltons.

Specifically, the polyethylene glycol-modified IL-2 described above can be prepared (and isolated) by a reaction of a polyethylene glycol modifier with an IL-2 protein (particularly, a certain molar ratio of the polyethylene glycol modifier to the IL-2 protein), for example, a shaking reaction in a pH neutral buffer at room temperature.

Preferably, the molar ratio of the IL-2 protein to the polyethylene glycol modifier includes the range of 1:3 to 1:10, preferably the range of 1:5 to 1:6, such as 1:3, 1:4, 1:5, 1:5.1, 1:5.2, 1:5.3, 1:5.4, 1:5.5, 1:5.6, 1:5.7, 1:5.8, 1:5.9, 1:6, 1:7, 1:8, 1:9, or 1:10.

In some embodiments of the present disclosure, the polyethylene glycol modifier is 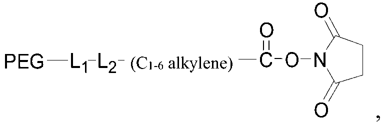 such as 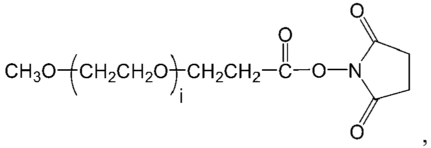 whose molecular weight may be 300-100000 daltons, preferably 10000-60000 daltons, such as 300, 500, 1000, 5000, 10000, 15000, 20000, 25000, 30000, 35000, 40000, 45000, 50000, 55000, 60000, 65000, 70000, 75000, 80000, 85000, 90000, 95000, or 100000 daltons.

Preferably, the radiation includes one or two or more of the following groups:
1) radiation produced by a natural radioisotope;
2) an X-ray; and/or
3) an electron beam, fast neutrons, a proton beam, a negative π-meson beam, or a heavy particle beam.

Preferably, the radiation produced by the natural radioisotope in 1) includes one or two or more of an α-ray, a β-ray, or a γ-ray.

Preferably, the radiation produced by the natural radioisotope in 1) is delivered via internal or external irradiation.

Preferably, the X-ray in 2), or the electron beam, the fast neutrons, the proton beam, the negative π-meson beam, or the heavy particle beam in 3) is delivered via external irradiation.

Preferably, the heavy particle beam includes, but is not limited to, heavy particle beams of helium, carbon, nitrogen, oxygen, neon, and the like.

In one specific embodiment of the present disclosure, the radiation is an X-ray.

Preferably, the polyethylene glycol-modified IL-2 is administered at a single dose of any value of 0.1 µg/kg-1000 mg/kg, preferably any value of 1 µg/kg-100 mg/kg, such as 0.1 µg/kg, 1 µg/kg, 10 µg/kg, 50 µg/kg, 100 µg/kg, 200 µg/kg, 300 µg/kg, 400 µg/kg, 500 µg/kg, 600 µg/kg, 700 µg/kg, 800 µg/kg, 900 µg/kg, 1 mg/kg, 10 mg/kg, 100 mg/kg, 200 mg/kg, 300 mg/kg, 400 mg/kg, 500 mg/kg, 600 mg/kg, 700 mg/kg, 800 mg/kg, 900 mg/kg, or 1000 mg/kg.

Preferably, the radiation is delivered at a dose of any value of 1-100 Gy/fraction, preferably any value of 1-60 Gy/fraction, and further preferably any value of 1-20 Gy/fraction, such as 1, 2, 3, 4, 5, 6, 7, 8, 9, 10, 15, 20, 25, 30, 35, 40, 45, 50, 55, 60, 65, 70, 75, 80, 85, 90, 95, or 100 Gy/fraction. Preferably, the number of fractions in which the radiation is delivered is any value of 1-50, preferably any value of 1-30, and further preferably any value of 1-10, such as 1, 2, 3, 4, 5, 6, 7, 8, 9, 10, 15, 20, 25, 30, 35, 40, 45, or 50.

It is well known that the dose of a drug used can be adjusted by the experience of a physician and the condition of a disease. The dose for humans and non-human animals can also be calculated according to conventional conversion forms in the prior art.

Preferably, the tumor includes a solid tumor and/or a non-solid tumor.

Preferably, the tumor includes a benign tumor or a malignant tumor.

Preferably, the tumor includes one or two or more of lymphoma, cervical cancer, leukemia, ovarian cancer, nasopharyngeal cancer, breast cancer, endometrial cancer, gastrointestinal tumor, bladder cancer, lung cancer, bronchial cancer, bone cancer, prostate cancer, renal cancer, thyroid cancer, head and neck cancer, testicular cancer, glioma, astrocytoma, skin cancer, myelodysplastic syndrome, or sarcoma.

Further preferably, the gastrointestinal tumor includes one or two or more of esophageal cancer, gastric cancer, liver cancer, colon cancer, rectal cancer, gallbladder cancer, or pancreatic cancer.

Further preferably, the skin cancer includes one or two or more of squamous cell carcinoma, basal cell carcinoma, melanoma, or eczematous carcinoma.

Further preferably, the lung cancer includes, but is not limited to, non-small cell lung cancer (e.g., Lewis lung cancer) or small cell lung cancer.

Further preferably, the glioma includes, but is not limited to, brain glioma or glioblastoma.

Further preferably, the leukemia includes, but is not limited to, one or two or more of acute lymphocytic leukemia, acute myelogenous leukemia, myelogenous leukemia, chronic lymphocytic leukemia, multiple myeloma, plasma cell leukemia, or chronic myelogenous leukemia.

Further preferably, the lymphoma includes, but is not limited to, Hodgkin lymphoma and non-Hodgkin lymphoma.

Further preferably, the lymphoma includes, but is not limited to, one or two or more of B-cell lymphoma (e.g., diffuse large B-cell lymphoma or marginal zone B-cell lymphoma), follicular lymphoma, mantle cell lymphoma, T-cell lymphoma, or Waldenström's macroglobulinemia.

Further preferably, the sarcoma includes, but is not limited to, one or two or more of osteosarcoma, Ewing's sarcoma, leiomyosarcoma, synovial sarcoma, soft tissue sarcoma, angiosarcoma, liposarcoma, fibrosarcoma, rhabdomyosarcoma, or chondrosarcoma.

In one specific embodiment of the present disclosure, the tumor includes one or two or more of breast cancer (particularly primary or metastatic breast cancer), lymphoma (particularly B-cell lymphoma), lung cancer (particularly non-small cell lung cancer, e.g., Lewis lung cancer), gastrointestinal tumor (particularly rectal cancer, colon cancer, or gastric cancer), skin cancer (particularly melanoma), or sarcoma.

In a second aspect of the present disclosure, provided is use of polyethylene glycol-modified IL-2 in combination with a lymphokine-activated killer cell (LAK cell) and radiation in preparing a product for treating a tumor.

Preferably, the polyethylene glycol-modified IL-2, the radiation, and the tumor are as defined in the first aspect of the present disclosure.

In a third aspect of the present disclosure, provided is a treatment method for a tumor.

The treatment method comprises administering to a subject in need an effective amount of polyethylene glycol-modified IL-2 in combination with radiation irradiation (preferably local irradiation).

Preferably, a dosage form of the polyethylene glycol-modified IL-2 may be an oral dosage form, such as a tablet (such as, but not limited to, a tablet, a pill, a powder, a granule, a capsule, a lozenge, a syrup, a liquid, an emulsion, and a suspension) and an injection (such as subcutaneous injection, intravenous injection, intramuscular injection, and intraperitoneal injection).

In one specific embodiment of the present disclosure, the dosage form of the polyethylene glycol-modified IL-2 is an injection.

Preferably, the radiation irradiation is radiotherapy.

In one specific embodiment of the present disclosure, the treatment method comprises administering to a subject in need an effective amount of polyethylene glycol-modified IL-2, and performing radiotherapy on the subject in need.

Preferably, the radiation includes one or two or more of the following groups:
1) radiation produced by a natural radioisotope;
2) an X-ray; and/or
3) an electron beam, fast neutrons, a proton beam, a negative π-meson beam, or a heavy particle beam.

Preferably, the radiation produced by the natural radioisotope in 1) includes one or two or more of an α-ray, a β-ray, or a γ-ray.

Preferably, the radiation produced by the natural radioisotope in 1) is delivered via internal or external irradiation.

Preferably, the X-ray in 2), or the electron beam, the fast neutrons, the proton beam, the negative π-meson beam, or the heavy particle beam in 3) is delivered via external irradiation.

Preferably, the heavy particle beam includes, but is not limited to, heavy particle beams of helium, carbon, nitrogen, oxygen, neon, and the like.

In one specific embodiment of the present disclosure, the radiation is an X-ray.

Preferably, the polyethylene glycol-modified IL-2 is administered at a single dose of any value of 0.1 µg/kg-1000 mg/kg, preferably any value of 1 µg/kg-100 mg/kg, such as 0.1 µg/kg, 1 µg/kg, 10 µg/kg, 50 µg/kg, 100 µg/kg, 200 µg/kg, 300 µg/kg, 400 µg/kg, 500 µg/kg, 600 µg/kg, 700 µg/kg, 800 µg/kg, 900 µg/kg, 1 mg/kg, 10 mg/kg, 100 mg/kg, 200 mg/kg, 300 mg/kg, 400 mg/kg, 500 mg/kg, 600 mg/kg, 700 mg/kg, 800 mg/kg, 900 mg/kg, or 1000 mg/kg.

Preferably, the radiation is delivered at a dose of any value of 1-100 Gy/fraction, preferably any value of 1-60 Gy/fraction, and further preferably any value of 1-20 Gy/fraction, such as 1, 2, 3, 4, 5, 6, 7, 8, 9, 10, 15, 20, 25, 30, 35, 40, 45, 50, 55, 60, 65, 70, 75, 80, 85, 90, 95, or 100 Gy/fraction. Preferably, the number of fractions in which the radiation is delivered is any value of 1-50, preferably any value of 1-30, and further preferably any value of 1-10, such as 1, 2, 3, 4, 5, 6, 7, 8, 9, 10, 15, 20, 25, 30, 35, 40, 45, or 50.

Preferably, a treatment cycle of the treatment method includes:
1) polyethylene glycol-modified IL-2, once a week or once every two weeks, for 2-4 weeks;
2) radiation irradiation, five fractions per week or three fractions per week (with a one-day interval between fractions) or one fraction per week, for 2-3 weeks.

Preferably, the treatment method further comprises combined use with other tumor treatment methods, such as combined use with chemotherapy and a LAK cell.

The polyethylene glycol-modified IL-2, the radiation, and the tumor are as defined in the first aspect of the present disclosure.

The subject described in the present disclosure may be a human or non-human animal, and the non-human animal includes monkeys, pigs, cattle, horses, sheep, dogs, cats, mice, etc.

The polyethylene glycol-modified IL-2 described in the present disclosure is the same as PEG-IL2; abbreviations for IL-2 modified with different numbers of PEG are represented as follows: IL-2 modified with 1 PEG is abbreviated as 1PEG-IL2, IL-2 modified with 2 PEGs is abbreviated as 2PEG-IL2, and so on.

The term "alkyl" of the present disclosure refers to a hydrocarbon chain radical that is linear or branched and that does not contain unsaturated bonds, and that is attached to the rest of the molecule via a single bond. Typical alkyl groups contain 1 to 12 (e.g., 1, 2, 3, 4, 5, 6, 7, 8, 9, 10, 11, or 12) carbon atoms, preferably 1 to 6 carbon atoms; examples include methyl, ethyl, n-propyl, isopropyl, n-butyl, isobutyl, tert-butyl, n-pentyl, isopentyl, neopentyl, tert-pentyl, n-hexyl, isohexyl, etc.

The term "alkylene" refers to hydrocarbyl (divalent alkyl) formed from an alkane molecule by losing two hydrogen atoms, which may be linear or branched and is attached to the rest of the molecule via a single bond. Typical alkylene contains 1 to 12 (e.g., 1, 2, 3, 4, 5, 6, 7, 8, 9, 10, 11, or 12) carbon atoms, preferably 1 to 6 carbon atoms; examples include methylene (-CH₂-), ethylidene (-CH₂CH₂-), propylene (-CH₂CH₂CH₂-, -CH(CH₃)CH₂-, -CH₂CH(CH₃)-), butylene, and the like.

The term "comprises", "comprising", or "includes" described in the present disclosure is an openended description that includes the specified ingredients or steps as described, as well as other specified ingredients or steps that do not substantially affect the technical effect.

The term "and/or" described in the present disclosure encompasses all combinations of the items connected by the term, and each combination shall be deemed to have been individually listed herein. For example, "A and/or B" encompasses "A", "A and B", and "B". For another example, "A, B, and/or C" encompasses "A", "B", "C", "A and B", "A and C", "B and C", and "A and B and C".

The "effective amount" described in the present disclosure refers to an amount or dose that provides the desired treatment after being administered to a patient or an organ in a single dose or multiple doses, e.g., an amount or dose of polyethylene glycol-modified IL-2, and/or a dose of radiation irradiation.

The term "treat", "treating", or "treatment" described in the present disclosure refers to slowing, interrupting, arresting, controlling, stopping, reducing, or reversing the progression or severity of a sign, symptom, disorder, condition, or disease after the disease has begun to progress, but it does not necessarily involve the complete elimination of all disease-related signs, symptoms, conditions, or disorders.

### BRIEF DESCRIPTION OF THE DRAWINGS

Hereinafter, examples of the present disclosure will be described in detail with reference to the drawings, where:
FIG. 1 shows the HPLC assay spectrum of PEGylation reaction products.
FIG. 2 shows the CM chromatography separation spectrum of PEGylation reaction products.
FIG. 3 shows the purity analysis spectrum of the 2PEG-IL2 sample.
FIG. 4 is a graph showing the change in colon cancer tumor volume of mice in each group.
FIG. 5 is a graph showing the change in lung cancer tumor volume of mice in each group.
FIG. 6 is a graph showing the change in lymphoma tumor volume of mice in each group.

### DETAILED DESCRIPTION

The technical solutions in the examples of the present disclosure will be described clearly and completely below with reference to the drawings. It is obvious that the described examples are only a part of the examples of the present disclosure, rather than all of them. Based on the examples of the present disclosure, all other examples obtained by those of ordinary skill in the art without creative efforts shall fall within the protection scope of the present disclosure.

### Example 1: Preparation of PEGylation Reaction Mixture

An appropriate amount of an IL-2 protein (provided by Beijing JenKem Technology Co., Ltd.) was taken and exchanged into a 5 mM PB buffer at pH 7.0, and a predetermined amount of a PEG modifier (provided by Beijing JenKem Technology Co., Ltd.) was added according to a certain reaction molar ratio (IL2 protein:PEG modifier = 1:5.3). The resulting mixture was mixed uniformly and reacted at room temperature for 2 h with shaking. After the reaction was completed, the preparation of the reaction mixture was analyzed by HPLC.

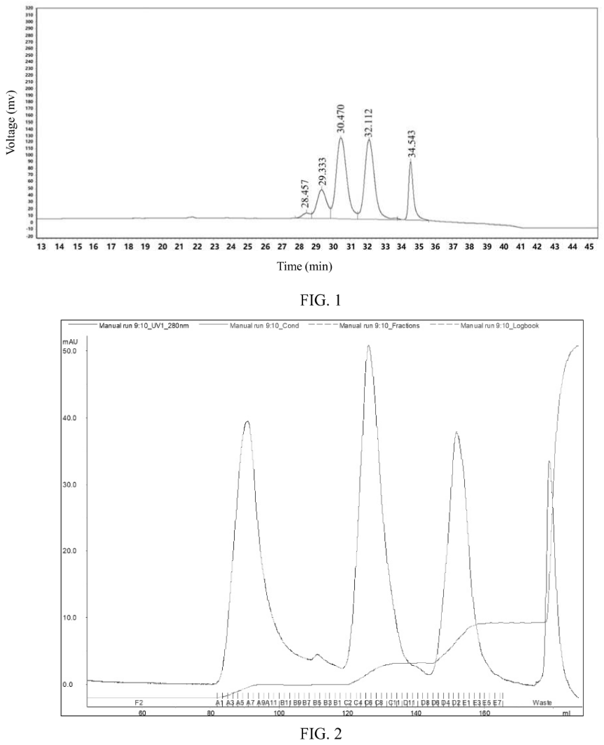

After the PEGylation reaction was completed, the content of each component was determined by HPLC analysis. The results are shown in Table 1 below and FIG. 1, where 2PEG-IL2 accounted for about 37%.

The structure of the used PEG modifier is shown as follows, with a molecular weight of 20000 daltons:

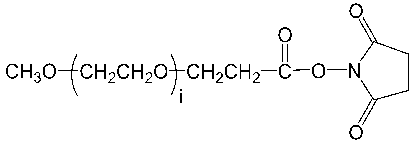

**Table 1: Composition analysis of reaction mixture (UV-0217-0002)**

| Peak number | Peak name | Retention time | Peak height | Peak area | Content% |
|---|---|---|---|---|---|
| 1 | 4PEG-IL2 | 28.457 | 7663.378 | 259121.828 | 1.8749 |
| 2 | 3PEG-IL2 | 29.333 | 43000.484 | 1744956.750 | 12.6260 |
| 3 | 2PEG-IL2 | 30.470 | 121325.367 | 5250551.500 | 37.9913 |
| 4 | 1PEG-IL2 | 32.112 | 118941.758 | 4828735.000 | 34.9392 |
| 5 | IL-2 | 34.543 | 86735.430 | 1737025.500 | 12.5686 |

### Example 2: CM Chromatography Method for Isolating 2PEG-IL2

(1) A 10 mM sodium acetate buffer at pH 3.0 was used as phase A, and a 10 mM sodium acetate buffer at pH 3.0 containing 1 M NaCl was used as phase B.
(2) The reaction mixture obtained in Example 1 was diluted with water while the pH was adjusted to 3.0 and the conductivity to 1.5 mS/cm, loaded onto an equilibrated CM column using an AKTA chromatography system, and subjected to step gradient elution with Phase B. The eluted fractions at a phase B concentration of 8% were collected and subjected to purity analysis by HPLC, to obtain the 2PEG-IL2 sample.
(3) The isolated 2PEG-IL2 sample was exchanged into a 5 mM PB buffer at pH 7.0 using a G25 desalting column for later use.

The CM chromatography separation spectrum is shown in FIG. 2.

Using a CM ion exchange column chromatography resin, samples conjugated with different numbers of PEGs were separated by step elution with increasing salt concentration. The peaks from left to right are sequentially >3PEG-IL2 (e.g., 3PEG-IL2 and 4PEG-IL2), 2PEG-IL2, 1PEG-IL2, and IL-2; the purity of the samples in different collection tubes was determined in combination with the HPLC assay method. The 2PEG-IL2 sample is mainly concentrated in the second peak from the left.

### Example 3: Alpha Affinity Chromatography

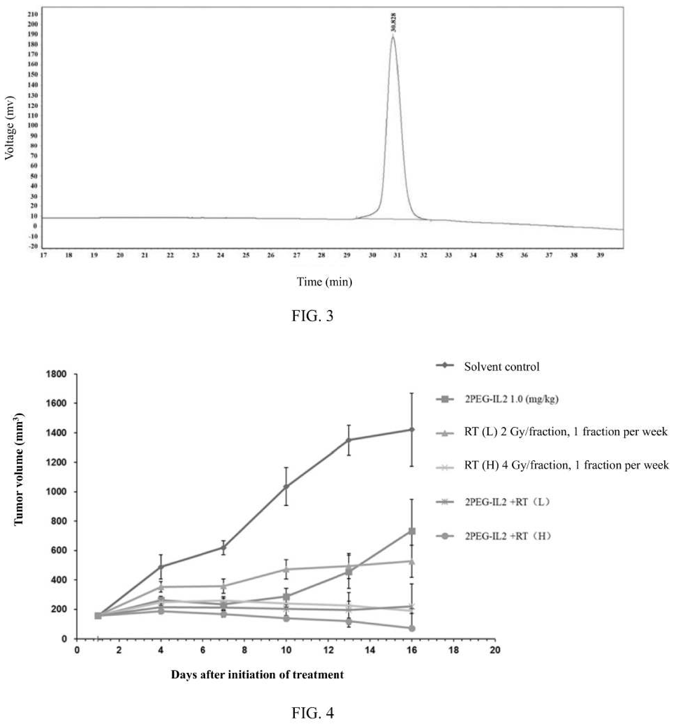

(1) A filler was equilibrated with a 5 mM PB buffer at pH 7.0.
(2) The sample obtained in Example 2 was loaded into the affinity filler.
(3) Elution was performed with a prepared eluent, and the elution sample was collected.
(4) The collected elution sample was exchanged into a 5 mM PB buffer at pH 7.0 using a G25 desalting column.
(5) The sample obtained in the previous step was concentrated, filtered through a 0.22 µm filter membrane for sterilization, and analyzed for purity by HPLC to obtain the final desired 2PEG-IL2. The purity analysis spectrum of the 2PEG-IL2 sample is shown in FIG. 3.

### Example 4: Effect of Combined Use of 2PEG-IL2 and Radiation Therapy on CT26 Cell Mouse Xenograft Tumor Model

1. CT26 cells (colon cancer cells) were cultured in an RPMI-1640 culture medium to the logarithmic growth phase, digested with trypsin, centrifuged, and adjusted to a cell concentration of 5 × 10⁶/mL with a PBS buffer.
2. 100 Balb/c mice were taken and inoculated with 0.1 mL of tumor cell fluid subcutaneously on the right back of each mouse, and the mice were bred in an SPF clean room. When the tumors grew to 100-150 mm³, the animals were randomly divided into 6 groups, with 10 in each group. The specific grouping and administration modes are shown in Table 2 below, where the solvent control group was given normal saline, and radiation therapy was performed via localized external irradiation of X-rays to the mouse tumor using an irradiator.

**Table 2: Experimental groups and administration modes**

| Group | | Number of animals | Dose (mg/kg) | Administration volume (mL/kg) | Frequency and route | Administration cycle |
|---|---|---|---|---|---|---|
| 1 | Solvent control | 10 | - | 5 | IV, QW | 4w |
| 2 | 2PEG-IL2 | 10 | 1.0 (mg/kg) | 5 | IV, Q2W | 4w |
| 3 | RT (L) | 10 | 2 Gy/fraction, 1 fraction per week | - | QW | 3w |
| 4 | RT (H) | 10 | 4 Gy/fraction, 1 fraction per week | - | QW | 3w |
| 5 | 2PEG-IL2 +RT (L) | 10 | 2PEG-IL2 (1.0mg/kg) | 5 | 2PEG-IL2, IV, Q2W | 4w |
| | | | RT (2 Gy/fraction, 1 fraction per week) | | QW | 3w |
| 6 | 2PEG-IL2 +RT (H) | 10 | 2PEG-IL2 (1.0mg/kg) | 5 | 2PEG-IL2, IV, Q2W | 4w |
| | | | RT (4 Gy/fraction, 1 fraction per week) | | QW | 3w |

| | | | | | | |
|---|---|---|---|---|---|---|
| RT: radiation therapy; H: high dose (high); L: low dose (low); IV: intravenous injection; QW: once a week; Q2W: once every two weeks. | | | | | | |

3. The growth of the tumors was dynamically observed, and the mice were weighed during the experiment. The long diameter (a) and the short diameter (b) of the tumors were measured by a vernier caliper. The tumor-bearing mice were sacrificed at the end of the experiment, and the tumor bodies were stripped. The stripped tumors were cleaned of bloodstains, fat, and other non-tumor tissues, and the tumor tissues were separately preserved in liquid nitrogen and 4% paraformaldehyde. The tumor volume and tumor volume inhibition rate were calculated according to the formula: mean tumor volume (mm³) = ab²/2; tumor volume inhibition rate (%) = (1 - mean tumor volume in experimental group/mean tumor volume in negative control group) × 100%.

### Experimental results:

The changes in tumor volume of the mice in each group are shown in FIG. 4, and the tumor growth inhibition rates of the mice in each group on day thirteen after treatment are shown in Table 3 below.

**Table 3: Tumor growth inhibition rates of mice in each group on day thirteen**

| Group | Tumor growth inhibition rate (%) |
|---|---|
| Solvent control | - |
| 2PEG-IL2 | 66.28 |
| RT (L) | 63.95 |
| RT (H) | 83.72 |
| RT (L)+2PEG-IL2 | 84.88 |
| RT (H)+2PEG-IL2 | 90.70 |

Analysis from the perspective of efficacy: (1) During the experiment, the tumor volume of the mice in the solvent control group showed a gradual upward trend, indicating that the mouse colon cancer model was successfully established and could be used for efficacy evaluation. The tumor growth rates of all treatment groups were slower than that of the solvent control group, indicating that these treatment modalities can inhibit tumor growth. (2) On day 13 after administration and treatment, the tumor volume of the mice in the combination group of 2PEG-IL2 and radiotherapy was significantly smaller than that in the 2PEG-IL2 alone group and the radiotherapy alone group, the tumor growth inhibition rate of the combination group of 2PEG-IL2 and radiotherapy was significantly increased compared with that of the 2PEG-IL2 alone group and the radiotherapy alone group, and the tumor growth inhibition effect exhibited a significant dose-effect relationship with the dose of radiotherapy. The results described above show that the antitumor efficacy of the combination of 2PEG-IL2 and radiotherapy is significantly superior to that of the two treatment modalities used alone.

Analysis from the perspective of safety: Radiotherapy only acts locally on tumors and causes relatively minor damage to other tissues and organs. 2PEG-IL2 can regulate the systemic immune system, and can effectively prevent tumor cell metastasis while killing tumor tissues.

### Example 5. Effect of Combined Use of 2PEG-IL2 and Radiotherapy on LLC Mouse Xenograft Tumor Model

1. Balb/c mice were inoculated with LLC cells (mouse lung cancer cells) in the axilla to establish a subcutaneous xenograft tumor model, with the cell inoculation amount being 5 × 10⁵ cells/mouse.
2. The mice were grouped for administration when the tumor volume reached 100-200 mm³.
3. 2PEG-IL2 was injected into the tail vein, the solvent control group was given normal saline, and radiotherapy was performed via localized external irradiation to the mouse tumor using an irradiator.
4. The state of the mice was observed periodically, the mice were weighed, and the tumor volume was measured.

The specific grouping and administration modes are shown in Table 4 below, where X-rays were used for radiation therapy:

**Table 4: Experimental groups and administration modes**

| | Group | Number of animals | Dose (mg/kg) | Administration volume (mL/kg) | Frequency and route | Administration cycle |
|---|---|---|---|---|---|---|
| 1 | Solvent control | 10 | - | 5 | IV, QW | 4w |
| 2 | 2PEG-IL2 | 10 | 1.0 (mg/kg) | 5 | IV, Q2W | 4w |
| 3 | RT (L) | 10 | 2 Gy/fraction, 1 fraction per week | - | 1 fraction per week | 3w |
| 4 | RT (H) | 10 | 4 Gy/fraction, 1 fraction per week | - | 1 fraction per week | 3w |
| 5 | 2PEG-IL2 +RT (L) | 10 | 2PEG-IL2 (1.0mg/kg) | 5 | IV, Q2W | 4w |
| | | | RT (2 Gy/fraction, 1 fraction per week) | | 1 fraction per week | 3w |
| 6 | 2PEG-IL2 +RT (H) | 10 | 2PEG-IL2 (1.0mg/kg) | 5 | IV, Q2W | 4w |
| | | | RT (4 Gy/fraction, 1 fraction per week) | | 1 fraction per week | 3w |

| | | | | | | |
|---|---|---|---|---|---|---|
| RT: radiation therapy; H: high dose (high); L: low dose (low); IV: intravenous injection; QW: once a week; Q2W: once every two weeks. | | | | | | |

### Experimental results:

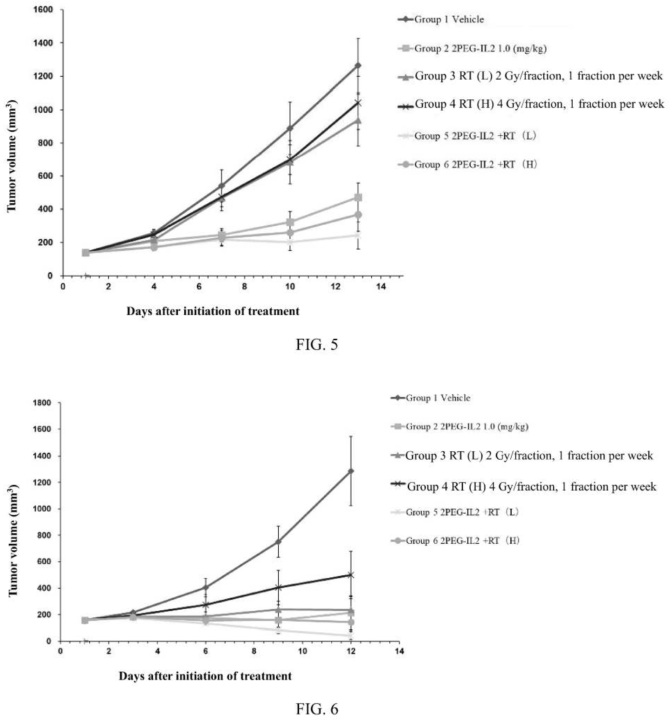

The changes in tumor volume of the mice in each group are shown in FIG. 5, and the tumor growth inhibition rates of the mice in each group on day thirteen after treatment are shown in Table 5 below.

**Table 5. TGI values of LLC mouse model on day 13 after grouping and administration**

| Group | TGI (D13) |
|---|---|
| Solvent control | - |
| 2PEG-IL2 | 62.72% |
| RT (L) | 25.77% |
| RT (H) | 17.60% |
| 2PEG-IL2 +RT (L) | 80.92% |
| 2PEG-IL2 +RT (H) | 70.90% |

Analysis from the perspective of efficacy: (1) During the experiment, the tumor volume of the mice in the solvent control group showed a gradual upward trend, indicating that the mouse lung cancer model was successfully established and could be used for efficacy evaluation. The tumor growth rates of all treatment groups were slower than that of the solvent control group, indicating that these treatment modalities can inhibit tumor growth. (2) On day 13 after administration and treatment, the tumor volume of the mice in the combination group of 2PEG-IL2 and radiotherapy was significantly smaller than that in the 2PEG-IL2 alone group and the radiotherapy alone group, and the tumor growth inhibition rate of the combination group of 2PEG-IL2 and radiotherapy was significantly increased compared with that of the 2PEG-IL2 alone group and the radiotherapy alone group. The results described above show that the antitumor efficacy of the combination of 2PEG-IL2 and radiotherapy is significantly superior to that of the two treatment modalities used alone. Analysis from the perspective of safety: Radiotherapy only acts locally on tumors and causes relatively minor damage to other tissues and organs. 2PEG-IL2 can regulate the systemic immune system, and can effectively prevent tumor cell metastasis while killing tumor tissues.

### Example 6: Effect of Combined Use of 2PEG-IL2 and Radiotherapy on A20 Mouse Xenograft Tumor Model

1. Balb/c mice were inoculated with A20 cells (mouse lymphoma cells) in the axilla to establish a subcutaneous xenograft tumor model, with the cell inoculation amount being 5 × 10⁵ cells/mouse.
2. The mice were grouped for administration when the tumor volume reached 100-200 mm³.
3. 2PEG-IL2 was injected into the tail vein, the solvent control group was given normal saline, and radiotherapy was performed via localized external irradiation to the mouse tumor using an irradiator.
4. The state of the mice was observed periodically, the mice were weighed, and the tumor volume was measured.

The specific grouping and administration modes are shown in Table 6 below, where X-rays were used for radiation therapy:

**Table 6: Experimental groups and administration modes**

| | Group | Number of animals | Dose (mg/kg) | Administration volume (mL/kg) | Frequency and route | Administration cycle |
|---|---|---|---|---|---|---|
| 1 | Solvent control | 10 | - | 5 | IV,QW | 4w |
| 2 | 2PEG-IL2 | 10 | 1.0 (mg/kg) | 5 | IV, Q2W | 4w |
| 3 | RT (L) | 10 | 2 Gy/fraction, 1 fraction per week | - | 1 fraction per week | 3w |
| 4 | RT (H) | 10 | 4 Gy/fraction, 1 fraction per week | - | 1 fraction per week | 3w |
| 5 | 2PEG-IL2 +RT (L) | 10 | 2PEG-IL2 (1.0mg/kg) | 5 | IV, Q2W | 4w |
| | | | RT (2 Gy/fraction, 1 fraction per week) | | 1 fraction per week | 3w |
| 6 | 2PEG-IL2 +RT (H) | 10 | 2PEG-IL2 (1.0mg/kg) | 5 | IV, Q2W | 4w |
| | | | RT (4 Gy/fraction, 1 fraction per week) | | 1 fraction per week | 3w |

| | | | | | | |
|---|---|---|---|---|---|---|
| RT: radiation therapy; H: high dose (high); L: low dose (low); IV: intravenous injection; QW: once a week; Q2W: once every two weeks. | | | | | | |

### Experimental results:

The changes in tumor volume of the mice in each group are shown in FIG. 6, and the tumor growth inhibition rates of the mice in each group on day twelve after treatment are shown in Table 7 below.

**Table 7. TGI values of A20 mouse model on day 12 after grouping and administration**

| Group | TGI (D12) |
|---|---|
| Solvent control | - |
| 2PEG-IL2 | 83.42% |
| RT (L) | 81.71% |
| RT (H) | 61.17% |
| 2PEG-IL2 +RT (L) | 96.73% |
| 2PEG-IL2 +RT (H) | 88.79% |

Analysis from the perspective of efficacy: (1) During the experiment, the tumor volume of the mice in the solvent control group showed a gradual upward trend, indicating that the mouse lymphoma model was successfully established and could be used for efficacy evaluation. The tumor growth rates of all treatment groups were slower than that of the solvent control group, indicating that these treatment modalities can inhibit tumor growth. (2) On day 12 after administration and treatment, the tumor volume of the mice in the combination group of 2PEG-IL2 and radiotherapy was significantly smaller than that in the 2PEG-IL2 alone group and the radiotherapy alone group, and the tumor growth inhibition rate of the combination group of 2PEG-IL2 and radiotherapy was significantly increased compared with that of the 2PEG-IL2 alone group and the radiotherapy alone group. The results described above show that the antitumor efficacy of the combination of 2PEG-IL2 and radiotherapy is significantly superior to that of the two treatment modalities used alone. Analysis from the perspective of safety: Radiotherapy only acts locally on tumors and causes relatively minor damage to other tissues and organs. 2PEG-IL2 can regulate the systemic immune system, and can effectively prevent tumor cell metastasis while killing tumor tissues.

The preferred embodiments of the present disclosure are described in detail above. However, the present disclosure is not limited to the specific details in the embodiments described above. Within the scope of the technical concept of the present disclosure, various simple modifications can be made to the technical solutions of the present disclosure, and all such simple modifications will fall within the protection scope of the present disclosure.

In addition, it should be noted that the various specific technical features described in the specific embodiments described above can be combined in any suitable manner provided that no contradiction arises. In order to avoid unnecessary repetition, various possible combinations will not be illustrated separately in the present disclosure.

## Claims

1. Use of polyethylene glycol-modified IL-2 in combination with radiation in preparing a product for treating a tumor.

2. The use according to claim 1, wherein the polyethylene glycol-modified IL-2 has a structure of formula (I) as follows: 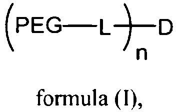
wherein PEG represents a polyethylene glycol residue;
L represents a linking group between PEG and D;
D represents a residue of IL-2;
n is an integer of 1-12; preferably, n is an integer of 1-7.

3. The use according to any one of claims 1-2, wherein L has the following structure: 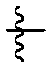 L₁-L₂-L₃ 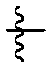 ,
wherein L₁ is selected from: a single bond, C1-6 alkylene, -(C1-6 alkylene)-NR^{L1}-, -(C1-6 alkylene)-O-, -(C1-6 alkylene)-S-, -(C1-6 alkylene)-NR^{L1}CO-, -(Cl-6 alkylene)-CONR^{L1}-, -(C1-6 alkylene)-CO-, -(C1-6 alkylene)-OCO-, -(C1-6 alkylene)-NHCONR^{L1}-, -(C1-6 alkylene)-SO₂-, and -(C1-6 alkylene)-SO-; L₂ is selected from: a single bond, C1-6 alkylene, cycloalkylene, arylene, and heterocyclylene; L₃ is selected from: C1-6 alkylene, -(C1-6 alkylene)-NR^{L2}-, -(C1-6 alkylene)-NR^{L2}CO-, -(C1-6 alkylene)-CO-, and -(C1-6 alkylene)-OCO-; R^{L1} and R ^{L2} are independently selected from: H and C1-6 alkyl;
preferably, L₃ is -(C1-6 alkylene)-CO-;
more preferably, L is -(C1-6 alkylene)-CO-.

4. The use according to any one of claims 1-3, wherein PEG has a molecular weight of 300-100000 daltons, preferably of 10000-60000 daltons;
preferably, PEG has a structure represented by formula (II) as follows: 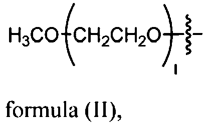
wherein i is an integer of 5-2500; or
PEG has a structure represented by formula (III) or formula (IV) as follows: 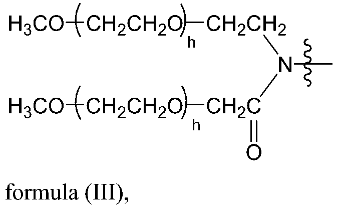 or 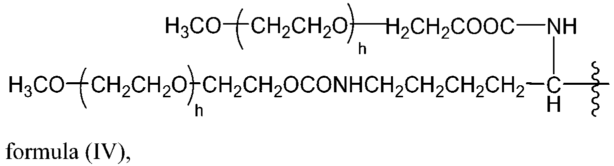
wherein h is an integer of 3-1200; or
PEG has a structure represented by formula (V) as follows: 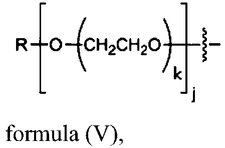
wherein:
k is an integer of 1-800;
j is an integer of 3-12;
R is a core molecule of multi-branched polyethylene glycol, and R may be selected from: residues of pentaerythritol, methyl glucoside, sucrose, diethylene glycol, propylene glycol, glycerol, and polyglycerol;
more preferably, PEG has a structure represented by formula (VI) as follows: 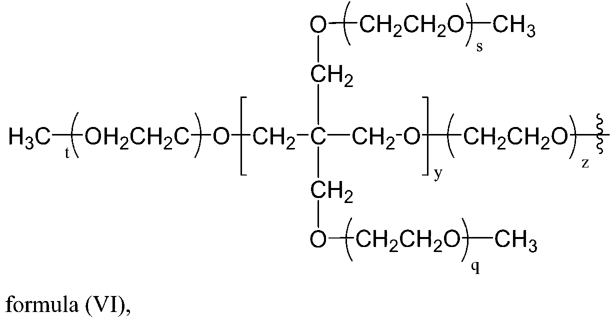
wherein q, s, t, and z are independently selected from an integer of 2-340,
and y is an integer of 1-5; or
PEG has a structure represented by formula (VII) as follows: 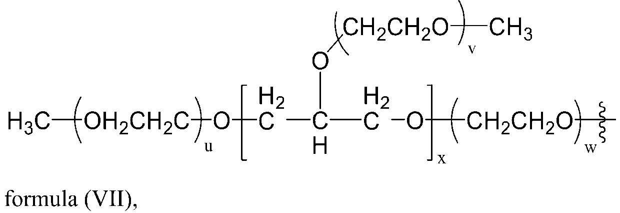
wherein u, v, and w are independently selected from an integer of 2-460,
and x is an integer of 1-5.

5. The use according to any one of claims 1-4, wherein the polyethylene glycol-modified IL-2 has a structure of formula (VIII) as follows: 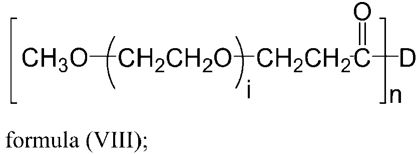 preferably, n is 1, 2, 3, or 4.

6. The use according to any one of claims 1-5, wherein the IL-2 is native IL-2, recombinant IL-2, or a mutant also having a native IL-2 function.

7. The use according to any one of claims 1-6, wherein a linking site of a polyethylene glycol modification on the IL-2 is an amino group, preferably, the linking site on the IL-2 comprises one or two or more of an N-terminal amino group of a peptide chain, a side chain amino group of a lysine residue within a peptide chain, a side chain amino group of an arginine residue, a secondary amine group on an imidazole ring of a histidine residue, and a secondary amine group on an indole ring of a tryptophan residue, and further preferably, the linking site on the IL-2 is a side chain amino group of a lysine residue within a peptide chain;
preferably, the polyethylene glycol modification is performed on a binding site in the IL-2 that binds to an IL-2 receptor α subunit.

8. The use according to any one of claims 1-7, wherein the radiation comprises one or two or more of the following groups:
1) radiation produced by a natural radioisotope;
2) an X-ray; and/or
3) an electron beam, fast neutrons, a proton beam, a negative π-meson beam, or a heavy particle beam.

9. The use according to claim 8, wherein the radiation produced by the natural radioisotope in 1) comprises one or two or more of an α-ray, a β-ray, or a γ-ray.

10. The use according to claim 8 or 9, wherein the radiation produced by the natural radioisotope in 1) is delivered via internal or external irradiation.

11. The use according to claim 8, wherein the X-ray in 2), or the electron beam, the fast neutrons, the proton beam, the negative π-meson beam, or the heavy particle beam in 3) is delivered via external irradiation.

12. The use according to any one of claims 1-11, wherein the polyethylene glycol-modified IL-2 is administered at a single dose of 0.1 µg/kg-1000 mg/kg, preferably 1 µg/kg-100 mg/kg.

13. The use according to any one of claims 1-12, wherein the radiation is delivered at a dose of 1-100 Gy/fraction, preferably 1-60 Gy/fraction, and further preferably 1-20 Gy/fraction.

14. The use according to claim 13, wherein the radiation is delivered in 1-50 fractions, preferably 1-30 fractions, and further preferably 1-10 fractions.

15. The use according to any one of claims 1-14, wherein the tumor comprises a solid tumor and/or a non-solid tumor.

16. The use according to any one of claims 1-14, wherein the tumor comprises one or two or more of lymphoma, cervical cancer, ovarian cancer, nasopharyngeal cancer, breast cancer, endometrial cancer, gastrointestinal tumor, bladder cancer, lung cancer, bronchial cancer, bone cancer, prostate cancer, renal cancer, thyroid cancer, head and neck cancer, testicular cancer, glioma, astrocytoma, skin cancer, myelodysplastic syndrome, or sarcoma.

17. The use according to claim 16, wherein the gastrointestinal tumor comprises one or two or more of esophageal cancer, gastric cancer, liver cancer, colon cancer, rectal cancer, gallbladder cancer, or pancreatic cancer.

18. The use according to claim 16, wherein the lymphoma comprises one or two or more of B-cell lymphoma, follicular lymphoma, mantle cell lymphoma, T-cell lymphoma, or Waldenström's macroglobulinemia.

19. The use according to claim 16, wherein the lung cancer comprises non-small cell lung cancer or small cell lung cancer.

20. A treatment method for a tumor, comprising administering to a subject in need an effective amount of polyethylene glycol-modified IL-2 in combination with radiation irradiation.

21. The treatment method according to claim 20, wherein the radiation comprises one or two or more of the following groups:
1) radiation produced by a natural radioisotope;
2) an X-ray; and/or
3) an electron beam, fast neutrons, a proton beam, a negative π-meson beam, or a heavy particle beam.

22. The treatment method according to claim 21, wherein the radiation produced by the natural radioisotope in 1) comprises one or two or more of an α-ray, a β-ray, or a γ-ray.

23. The treatment method according to claim 21 or 22, wherein the radiation produced by the natural radioisotope in 1) is delivered via internal or external irradiation.

24. The treatment method according to claim 21, wherein the X-ray in 2), or the electron beam, the fast neutrons, the proton beam, the negative π-meson beam, or the heavy particle beam in 3) is delivered via external irradiation.

25. The treatment method according to any one of claims 20-24, wherein the polyethylene glycol-modified IL-2 is administered at a single dose of 0.1 µg/kg-1000 mg/kg, preferably 1 µg/kg-100 mg/kg.

26. The treatment method according to any one of claims 20-25, wherein the radiation is delivered at a dose of 1-100 Gy/fraction, preferably 1-60 Gy/fraction, and further preferably 1-20 Gy/fraction.

27. The treatment method according to claim 26, wherein the radiation is delivered in 1-50 fractions, preferably 1-30 fractions, and further preferably 1-10 fractions.

28. The treatment method according to any one of claims 20-27, wherein the tumor comprises a solid tumor and/or a non-solid tumor.

29. The treatment method according to any one of claims 20-27, wherein the tumor comprises one or two or more of lymphoma, cervical cancer, ovarian cancer, nasopharyngeal cancer, breast cancer, endometrial cancer, gastrointestinal tumor, bladder cancer, lung cancer, bronchial cancer, bone cancer, prostate cancer, renal cancer, thyroid cancer, head and neck cancer, testicular cancer, glioma, astrocytoma, skin cancer, myelodysplastic syndrome, or sarcoma.

30. The treatment method according to claim 29, wherein the gastrointestinal tumor comprises one or two or more of esophageal cancer, gastric cancer, liver cancer, colon cancer, rectal cancer, gallbladder cancer, or pancreatic cancer.

31. The treatment method according to claim 29, wherein the lymphoma comprises one or two or more of B-cell lymphoma, follicular lymphoma, mantle cell lymphoma, T-cell lymphoma, or Waldenström's macroglobulinemia.

32. The treatment method according to claim 29, wherein the lung cancer comprises non-small cell lung cancer or small cell lung cancer.
